# EUROPEAN PATENT APPLICATION

(11) **EP 4 220 165 A1**
(43) Date of publication of application: **02.08.2023**
(21) Application number: 22154160.0
(22) Date of filing: 31.01.2022
(51) Int. Cl.: G01N 33/543

(54) **METHOD AND DEVICE FOR THE DETECTION OF TRAUMATIC BRAIN INJURIES**

(71) Applicant: HES-SO Valais-Wallis, 1950 Sion (CH)
(72) Inventor: PFEIFER, Marc Emil, 6330 Cham (CH); JOVIC, Milica Slavisa, 1950 Sion (CH); PRIM, Denis Alexandre, 1950 Sion (CH)
(74) Representative: KATZAROV S.A.

(57) **Abstract**

The present invention relates to an analytical cartridge for sample processing and quantitative multi-analyte immunoassay and a Point-of-Care in vitro diagnostic (IVD) medical device using said cartridge, where the cartridge comprises a sample inlet port (201), at least one reagent retaining element (204), a mixing unit (206) where the sample and the reagents are combined, a sensor unit (207) comprising electrodes pre-functionalized with biomarker(s)-specific ligands, where the mixed solution is reacted with pre-functionalized ligands, a reagent reservoir unit (211) adapted to dispense an ECL buffer into the sensor unit prior measurement, and characterized in that the sensor unit is adapted to be powered by an electrical supply source adapted to apply a potential to the pre-functionalized electrodes and trigger an ECL reaction between the reagents producing an ECL signal to be acquired by a detector unit (130).

## Description

### Technical Field

The present invention relates to a method and a device for the detection of traumatic brain injuries and more particularly to an analytical method for the detection of traumatic brain injury biomarkers and to a point-of-care (POC) diagnostic device for the detection of traumatic brain injury biomarkers. More particularly, it relates to an analytical method and a point-of-care in-vitro diagnostic (IVD) device for the detection of one or multiple biomarkers in biological samples.

### Background of the art

Traumatic brain injury (TBI) is a major cause of death and disability worldwide, especially in elderly people, children, and young adults.

More particularly, TBI is an injury to the brain caused by an external force. TBI can be classified based on severity ranging from mild traumatic brain injury (mTBI) to severe traumatic brain injury, mechanism (closed or penetrating head injury), or other features (e.g., occurring in a specific location of the brain or over a widespread area). They can result in physical, cognitive, social, emotional, and behavioural symptoms, and outcomes can range from complete recovery to permanent disability or death.

The causes of TBI include falls, vehicle collisions and violence. Brain trauma occurs as a consequence of a sudden acceleration or deceleration within the cranium or by a complex combination of both movement and sudden impact. In addition to the damage caused at the moment of injury, a variety of events following the initial injury may result in further injury. These processes include alterations in cerebral blood flow and pressure within the skull. Some of the imaging techniques used for diagnosis include computed tomography (CT) and magnetic resonance imaging (MRI).

However, these imaging techniques necessitate to be carried out in specific locations such as hospitals or specialized imaging centers and require time and significant costs. There is therefore a need for a device and a method which would permit to diagnose mTBI or at least the severity of the TBI immediately (i.e., on-site or locally) after a shock without necessitating specialized users or particularly specialized centers.

Nowadays, biomarkers can be applied across several contexts of use, including diagnostic, prognostic, predictive, pharmacodynamic, efficacy response, and personalized medical applications. In the case of mild traumatic brain injury (mTBI) the most promising are brain protein biomarkers that can pass the blood brain barrier following the injury. These biomarkers can be detected in serum and/or plasma, and the analytical methods used are able to accurately quantify their concentrations on the order of tens of picograms per milliliter (pg mL⁻¹).

Different biomarkers have been proposed so far in the context of mTBI. For example, Posti et al. (J Neurotrauma 2019, 36, 2178-2189) and Lagerstedt et al. (PLOS ONE 2018, 13, e0200394) reported on panels of protein biomarkers that perform better in discriminating CT positive from CT negative patients with mTBI than individual biomarkers. Banyan Biomarkers proposed a core lab blood assay for measuring the concentrations of two proteins, glial fibrillary acidic protein (GFAP) and ubiquitin carboxyl-terminal hydrolase isozyme L1 (UCH-L1). However, despite of these developments in the mTBI diagnostic field, there is still a growing need to expand the biomarker panels with additional biomarkers or biomarker types like inflammatory proteins and brain damage proteins to assure sufficient clinical specificity and sensitivity in evaluating concussions in mTBI-patients. The ability to perform biomarker measurements anywhere and early (e.g., on site of the accident) as well as repeatedly to allow follow-up measurements for trending/monitoring and outcome assessment, thus in the patient proximity, at so-called "point-of-care (POC)" would be a game-changer in the sense that e.g., family doctors, nurses/physicians at hospital emergency rooms (ER), paramedics/first responders, etc. will be able to acquire clinically very useful (and early) physiological data. This would help a great deal with a better (TBI/mTBI) diagnosis, better treatment/therapy and decreasing the number of non-diagnosed individuals.

It is therefore an object of the invention to provide a POC diagnostic test device and methodology for (TBI/mTBI) biomarkers detection which will help to avoid unnecessary CT scans (cf. costs and harmful ionizing radiation), support rapid decentralized diagnostics, and allow a better and more personalized patient management. Such tests need to be fast (< 30 min), require miniaturized and portable (hand-held) devices that are expected to perform comparably to their central laboratory instrument analogues, and low-cost regarding reagents and consumables used.

Different POC instruments have been proposed in the context of TBI/mTBI biomarkers and were disclosed in US2009041830 AA and US2019053744 AA for example, but development of a sensitive, multiplexed POC diagnostic test remains challenging particularly when the volume of sample is limited, which is often the case for biological samples such as blood, serum, plasma, etc. A variety of analytical (detection) techniques have been employed, including fluorescence, electrochemistry, surface plasmon resonance, and chemiluminescence. However, some techniques require costly array detectors or light sources and are impacted by crosstalk, while the sensitivity may be insufficient for multiplex detection in clinical samples. Some approaches are little or not at all compatible with compactness (I.e., small size), weight (cf. hand-held application) and robustness requirements.

In this regard, a primary object of the invention is to solve the above-mentioned problems and more particularly to provide a POC TBI diagnostic device and methodology with high sensitivity for multiplex detection in clinical samples.

### Summary of the invention

The above problems are solved by the present invention which is a device that comprises a cartridge and a base station (also called device or reader) for the detection of biomarkers associated with traumatic brain injury (TBI). The cartridge and device may also constitute a platform that supports other POC diagnostic applications, e.g., in the field of cardiovascular diseases (CVD).

The present invention provides an in-vitro diagnostic (IVD) device specifically configured for multiplexed POC detection of biomarkers from a small volume of biological sample. It comprises a portable (hand-held) diagnostic device to measure biomarkers that are indicative of traumatic brain injury. Preferably, the biomarkers are proteins, fragments, or derivatives thereof, and are associated with neuronal cells, glial cells, other brain cells, or any cell that is present in the brain and the central nervous system (CNS).

Preferably, the POC in-vitro diagnostic (IVD) device performs sensitive and multiplexed detection of mild traumatic brain injury biomarker panel from a single sample with an electrochemiluminescence (ECL) detection method.

Indeed, ECL shows great promise due to excellent sensitivity and large dynamic range as well as good selectivity because excitation can be modulated by alternation of the applied potential, which allows greater control over emission location as ECL emission occurs close to the electrode surface that consequently facilitates detection of multiple analytes in the same sample using single or multiple electrodes. ECL as opposed to e.g., fluorescence does not require a light source or complex optics.

A first aspect of the invention is an analytical cartridge for sample processing and quantitative multi-analyte immunoassay comprising a sample inlet port, at least one reagent retaining element, a mixing unit where the sample and the reagents are combined, a sensor unit comprising electrodes pre-functionalized with biomarker(s)-specific ligands, where the mixed solution is reacted with pre-functionalized ligands, a reagent reservoir unit adapted to dispense an ECL buffer into the sensor unit prior measurement, and characterized in that the sensor unit is adapted to be powered by an electrical power supply source adapted to apply a potential to the pre-functionalized electrodes and trigger an ECL reaction between the reagents producing an ECL signal to be acquired by a detector unit.

Advantageously, the analytical cartridge is a cartridge for quantitative multi-analyte immunoassay for TBI and/or mTBI diagnostics.

Preferably, the sample inlet port is designed to collect a volume between 10 to 100 µL.

According to a preferred embodiment of the present invention, the reagent reservoir(s) are adapted to remove non-specifically bound compounds in the sensor unit and to dispense an ECL buffer into the sensor unit prior measurement.

Alternatively, the cartridge further comprises a wash buffer unit adapted to remove the non-specifically bound compounds in the sensor unit.

Advantageously, the cartridge further comprises a sample processing unit comprising at least one of a filtration unit or a microfluidic blood separator system, and a metering device adapted to support accurate quantification of the biomarkers, and comprising at least one of an active valve, a passive microfluidic channel, and/or a membrane.

In a preferable manner, at least one reagents reservoir is adapted to store reagents immobilized, lyophilized, or in solution.

According to a preferred embodiment of the present invention, one reagents reservoir is adapted to be actuated by an integrated pumping module or by applying manually or mechanically pressure on a pouch.

Preferably, the reagents solution stays in the sensor unit during an incubation time of between 1 to 30 min.

Advantageously, the cartridge comprises a transparent cartridge window.

A second aspect of the invention is a point-of-care in vitro diagnostic (IVD) medical device for quantitative multi-analyte immunoassay comprising the analytical cartridge of the first aspect and a base station, wherein the base station comprises a cartridge interface for connecting to the cartridge, electronics for managing the tasks of analysis/measurement and a user interface (UI) comprising one or multiple display(s) and buttons/keyboard/touch screen, actuators for activating the fluids in the cartridge through specific connectors on the device and on the cartridge, an electrochemistry unit comprising an electronic hardware employed for an ECL signal trigger in sensor unit of the cartridge, via an interface, said trigger unit comprising an electrical power source adapted to apply a potential to the sensor unit through the pre-functionalized electrodes and trigger an ECL reaction between the reagents, a detector unit for detecting ECL signal generated in the sensor unit of the cartridge composed of a detector and connected via an optic path to a window interfacing with the cartridge via a window on the cartridge, and a communication unit adapted to provide connectivity to the cartridge with an NFC reader to access cartridge data and ID and a Network (5G/WIFI/BT) unit for database connections, software updates and general communication.

A third aspect of the invention is a biomarker detection method comprising the steps of obtaining a biological sample from a human subject and performing the sample processing steps to assess TBI and mTBI biomarker(s) potentially present in the biological sample, binding of one or a plurality of TBI and mTBI biomarkers present in the biological sample with biomarker(s)-specific ligands coupled with an electrochemiluminescent detection label (L1/ECL) to form a biomarker(s)-L1/ECL complex(es), binding of said biomarker(s)-L1/ECL complex(es) in the sensor unit to the conductive electrode surface functionalized with a plurality of biomarker(s)-specific ligands (L2) to form a sandwich immunoassay complex(es) (L1/ECL-biomarker(s)-L2), wherein each of the ligands L2 can be immobilized at a different, separate locations on the conductive electrode surface, washing step, adding an ECL buffer solution, detecting of ECL signal for each biomarker by applying potential to the sensor unit to trigger the ECL read-out and correlating it to the concentration of TBI/mTBI biomarker(s) present in the biological sample.

Preferably, the electrode's surface can be functionalized with one or a plurality of biomarker(s) specific ligands (L2), on one or multiple, different, indexable, locations on the electrode.

According to a preferred embodiment of the present invention, electrochemiluminescent (ECL) detection labels are loaded on carrier particles, taken in the group comprising mesoporous, conductive, magnetic and nano/microparticles, wherein the carrier particles carrying the label are coupled to biomarker(s)-specific ligands (L1).

Advantageously, the electrode is modified with a multi-purpose functionalized interface layer wherein the layer is formed from various materials and exhibits and/or improves one or multiple properties, such as antifouling (reduces background interference and/or non-specific binding), enrichment (target analyte/antibody/ECL detection label enrichment and/or antigen antibody binding events/kinetics improvement), conductivity (improves electron transfer and ECL signal intensities) and wettability (improves wettability of the electrode and liquid flow).

A fourth aspect of the invention is a TBI and mTBI diagnosis method comprising the biomarker(s) detection method of the third aspect and further comprises the steps of determining whether the TBI and mTBI biomarker(s) concentrations are above or below a predetermined threshold range, based on the determining step, judging that TBI and mTBI is diagnosed if the calculated concentrations are above said threshold ranges, displaying results to the user for IVD TBI and mTBI diagnosis, saving results for kinetic (i.e., multiple follow-up) analyses (monitoring) of biomarkers over time with multiple sampling of the same patient. The evaluation of the kinetics can support the diagnosis of TBI or mTBI.

According to a preferred embodiment of the present invention, the method further comprises the step of the saving results in a patient's database comprising data corresponding to biomarker(s) measurements performed at different time points with respect to the target marker(s) and wherein the data and background information related to the patient comprises demographic information corresponding to at least one of age, race, gender, body mass index, and morbidities.

### Brief description of the drawings

Further particular advantages and features of the invention will become more apparent from the following non-limitative description of at least one embodiment of the invention which will refer to the accompanying drawings, wherein
- Figure 1 represents an embodiment of the point-of-care diagnostic device and cartridge components of the present invention and their functionalities;
- Figures 2A and 2B represent an embodiment of a cartridge integrated components and functionalities of the present invention, and view of relative positions of the cartridge components, respectively;
- Figures 3A and 3B schematically represent an embodiment of a multi-analyte sensor unit embedded in the cartridge of the present invention depicted in Figures 2A and 2B; and an example of an ECL bioassay performed in the multi-analyte sensor unit, respectively;
- Figure 4 is a flowchart schematically representing Main device workflow of the present invention;
- Figure 5 is a flowchart schematically representing Cartridge workflow according to the present invention including sample and reagent processing steps;
- Figure 6 is a flowchart schematically representing an ECL bioassay workflow according to the present invention;
- Figure 7 is a flowchart schematically representing the method steps and workflow carried out in the device of the present invention.

### Detailed description of the invention

The present detailed description is intended to illustrate the invention in a non-limitative manner since any feature of an embodiment may be combined with any other feature of a different embodiment in an advantageous manner.

The present invention relates to a method and in-vitro diagnostic POC system for sensitive detection of one or multiple TBI and/or mTBI biomarkers in biological samples and based on electrochemiluminescence method.

Examples of biological samples that may be used with the method and device described herein include blood, serum, plasma, urine, saliva, cerebrospinal fluid (CSF). These and other aspects are described further below with reference to the figures. Optional features of the invention are set out in the dependent claims.

First, we will describe the function and the structure of the device and the cartridge according to a preferred embodiment of the invention.

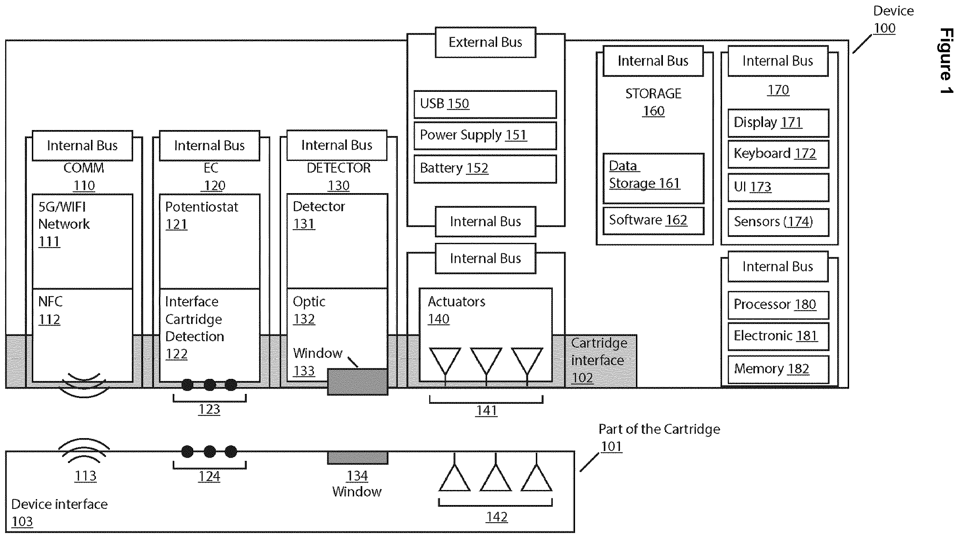

Figure 1 is a simplified schematic showing a preferred embodiment of the point-of-care diagnostic device 100 of the present invention connected to the cartridge 101 through a cartridge interface 102 of the device and the device interface 103 of the cartridge. Cartridge interface and Device interface are designed to be interconnected to allow electrical power and actuator pressure to be transferred from the device to the cartridge and an optic part to be aligned with the measurement sensor. The signal from the potentiostat could be, for instance, connected via a pin connector. The pressure to actuate reagents in the cartridge could be transferred via a pressure switch. The device 100 is composed of an electronic board 181 equipped with a processor 180 and memory 182 (RAM) for processing. The storage unit 160 is composed of a data storage 161 and the software 162 installed on it. A communication (COMM) unit 110 provides connectivity to the cartridge with an NFC reader 112 to access cartridge data and ID 113 and a Network (5G/WIFI/BT) 111 unit for database connection, software updates and general communication. The software 162 is installed on the storage unit 160 and manages the task of analysis/measurement and the user interface (UI) 173 comprising one or multiple display(s) 171 and buttons/keyboard/touch screen 172. The device is also equipped with a detector unit 130 for detecting ECL signals generated in the sensor unit of the cartridge. The detector unit 130 is composed of a detector 131 (e.g., charged-couple device (CCD), electron multiplication CCD (EMCCD) complementary metal-oxide semiconductor (CMOS), scientific complementary metal-oxide semiconductor (sCMOS) sensor, one or multiple photodiode array(s) (PDA), avalanche photodiodes (APD), photomultiplier tubes (PMT), or multi-pixel photon counters (MPPC)) and connected via an optic path 132 to a window 133 for interfacing with the cartridge 101 via a window on the cartridge 134. Electrical trigger for ECL signal is applied by an electronic hardware (potentiostat) 121 equipped on the EC (electrochemistry) unit 120 via an interface 122, 123 to the cartridge 124. The device can be connected to a computer via the USB Interface 150 or to a power supply 151 to recharge the integrated/replaceable battery 152. Fluids in the cartridge are activated by the device via dedicated actuators 140 through specific connectors on the device 141 and on the cartridge 142. These fluids are the reagents integrated to the cartridge that could comprise, but not limited to, buffers, wash buffers, ECL reading buffer and ligands solution, are activated, or pushed or moved, according to the method, in the cartridge by actuators. Actuators could be pressure switch or syringe for example. Actuators 140 can comprise a pump or a syringe pump, or a mechanically or manually ductile/compressible pouch or the fluids may be capillary force driven (e.g., with paper). The internal bus interconnects all subsystems of the device. Device integrated sensors 174 provide information on the status of the system (for instance, temperature, humidity, position, vibration) and are saved with each data for quality management (QM) purposes.

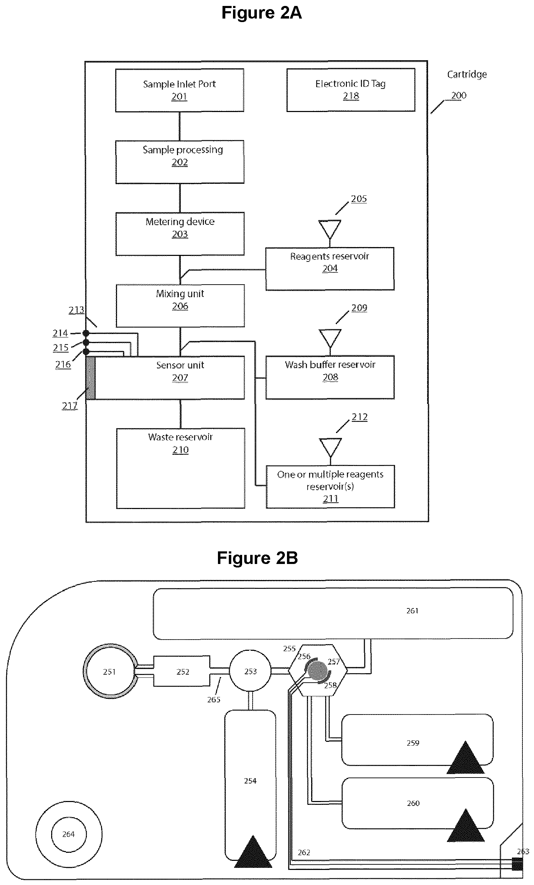

Figure 2A shows more detailed embodiment of the structure of the cartridge components and functionalities.

As shown the cartridge 200 preferably comprises a sample inlet port 201, a sample processing unit 202, a metering device 203, a reagent retaining element 204, such as a reservoir or a conjugate pad, a mixing unit 206, a sensor unit 207, a wash buffer reservoir 208, a waste reservoir 210, actuators 205, 209, and 212, and one or multiple reagents reservoirs 211.

The cartridge is preferably made of a plastic housing and can integrate the quantitative multi-analyte immunoassay specifically for TBI and mTBI diagnostics. The sample is introduced into the sample inlet port 201, this inlet is preferably designed to collect a volume between 10 to 100 uL and is compatible with, for instance, capillary collection tube or deposition of a drop of biological fluid (e.g., whole blood, serum, etc.) from a pipette. Plasma is then separated from whole blood in the sample processing unit 202 composed of for instance a filtration unit or a microfluidic blood separator system. The embedded metering device 203 is built along the channel to support accurate quantification of the biomarkers. It could be for instance composed of an active valve, a passive microfluidic channel, or a membrane. Lyophilized reagents or reagents in solution are stored in the integrated reagent retaining element(s) 204 and actuated by a device/reader integrated pumping module or by applying manually or mechanically pressure on a pouch 205. The reagent solution is combined (such as mixed or the same) with the sample in the mixing unit 206 that could be composed of a fluidic channel or a membrane. Furthermore, liquid flows in the sensor unit 207, described more in details below, composed of two- or three-electrode system (single, multiple electrodes or interdigitated electrodes) where the reaction with pre-functionalized reagents occurs. After an incubation time of between 1 to 30 min, the wash buffer 208 is actuated by 209 to remove non-specifically bound compounds in the sensor unit and the ECL buffer is then dispensed by (the actuator) 212 into the sensor unit from reservoir(s) 211 prior measurement. A potential is then applied through device embedded electrical power supply (EC unit 120, see Fig. 1) via the electric connection pads 213 to the sensor unit to trigger the ECL reaction between the reagents. The sensor unit is composed of a reference electrode connected by 214, a counter electrode connected by 215 and a working electrode connected by 216 and further described in Figure 2B. The reference electrode connected by 214 may be combined with the counter electrode connected by 215 to obtain a two-electrode system (working electrode and reference/counter electrode). Generated ECL signal is measured by the device embedded detector (Detector unit 130, see Fig. 1) through the transparent cartridge window 217. For quality management and unique identification reasons an electronic tag is embedded in each cartridge composed of a barcode (QR) and a RFID antenna 218.

Figure 2B shows an example of a single use (i.e., disposable) cartridge integrating the required components explained above for the detection of TBI/mTBI biomarkers in whole blood or serum. The sample is deposited in the sample inlet 251 and processed in the sample processing unit 252 composed for instance of a serum separation membrane or microfluidic driven separator. The biomarkers are then transported through a microfluidic or on a membrane 265 to a mixing unit (area) 253 and combined with the biomarker(s)-specific ligands (L1) coupled with electrochemiluminescent (ECL) detection label(s) stored in 254 and actuated by the device and software. The system monitors the reaction time according to the method and transports the solution to the sensors unit 255 where the biomarker(s)-specific ligands (L2) were immobilized on the working electrode 257. A washing step, actuated by the device and software, is performed with the wash buffer stored in a dedicated reservoir 260 and transported to the sensor unit 255 to remove all unreacted compounds. Once completed, ECL read buffer is delivered, actuated by the device and software, from one or multiple reservoirs (one is shown in the Figure 2B, 259) and transported to the sensor unit 255. The device and software trigger the potential from the integrated electrical power supply (e.g., potentiostat) (EC unit 120, see Figure 1) by the cartridge connectors pads 263 via electrical tracks 262 to, for instance, a three electrodes system (shown here) composed of the working electrode 257, the counter electrode 256, and the reference electrode 258. The excess of reagents and wash buffer is transported to the waste reservoir 261. The cartridge integrates an identification tag 264 composed, for instance, of an NFC chip.

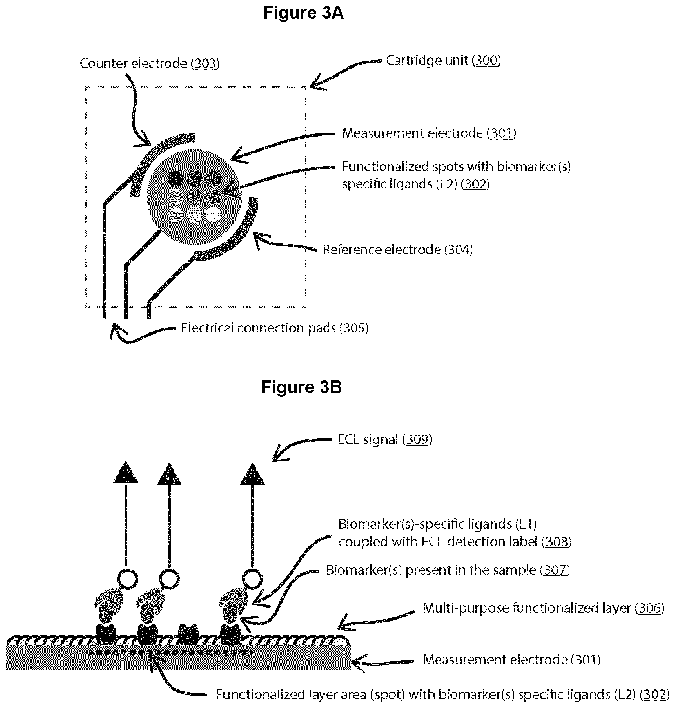

Figure 3A shows an example of a multi-analyte sensor unit for the detection of TBI/mTBI biomarker(s). The sensor unit embedded in the cartridge shown in Figure 2B can be composed of one or multiple working (measurement) electrode(s) 301, a counter electrode 303, and a reference electrode 304, all being connected to the electrical power supply (EC unit 120, see Fig. 1) via electrical connection pads 305. The reference electrode 304 usually is in its equilibrium state and has a high resistance and intrinsic electrode potential. It may be combined with the counter electrode 303 to obtain a two-electrode system (working electrode and reference/counter electrode).

Electrodes may be fabricated using various microfabrication techniques (e.g., lithography, inkjet printing, aerosol jet printing, roll-to-roll printing, screen-printing, etc.) from various materials (e.g., metals, metal oxides, carbon-based materials, polymers, etc.). Optionally, working electrode's surface 301 can be functionalized with one or plurality of biomarker(s) specific ligands (L2), on one or multiple, different, indexable, locations 302.

Figure 3B illustrates the formation of the ECL sandwich immunoassay complex(es) on one or multiple working (measurement) electrode(s) 301, formed between the biomarker(s)-specific ligands L2, 302 immobilized on the electrode(s), biomarker(s) present in the sample 307, and biomarker(s)-specific ligands (L1) coupled with electrochemiluminescent (ECL) detection label(s) L1/ECL, 308. Generation of ECL signal 309 is directly proportional to the amount of ECL label and therefore to the amount of biomarker(s) present in the sample 307. Alternatively, electrochemiluminescent (ECL) label(s) can be loaded on (a) carrier particle(s) (e.g., mesoporous, conductive, magnetic, nano or microparticle(s)), wherein the carrier particle(s) carrying the label may be coupled to biomarker(s)-specific ligands (L1). Without being bound by theory, it is believed that the use of carrier particle(s) can provide improved signal in the ECL bioassays in several ways: (i) by providing a scaffold with high surface area for multiple ECL labels; (ii) conductive carriers can conduct electrons from the working electrode so as to oxidize or reduce ECL labels on its surface; (iii) magnetic carrier can be used to bring the ECL labels closer to the working electrode (confinement); (iv) carrier particle may comprise a material capable of acting as a working electrode for inducing ECL from a certain ECL labels and a particular ECL co-reactant (e.g., the material is "ECL active").

The working electrode 301 may be also modified with a multi-purpose functionalized interface layer 306 wherein the layer may be formed from various materials and exhibits and/or improves one or multiple properties from the following list:
(a) antifouling (reduces background interference and/or non-specific binding) (e.g., hydrogels, polyethyleneglycols (PEG), bovine serum albumin (BSA), casein, Tween-20, etc.);
(b) enrichment (target analyte/antibody/ECL label enrichment and/or antigen-antibody binding events/kinetics improvement) (e.g., hydrogels, polymers, mesoporous particles, magnetic particles, ECL-active molecular moieties for luminescent (Cartesian) orientation points, etc.);
(c) conductivity (improves electron transfer and ECL signal intensities) (e.g., conductive polymers, CNTs, metallic NPs, or combinations thereof);
(d) wettability (improves wettability of the electrode and liquid flow) (e.g., hydrophilic materials, polymers, etc.).

We will now more specifically describe the different processes carried out in the context of the present invention.

While a general method is carried out by the device and the cartridge of the present invention, this general method may be divided in four workflows: the workflow of the device, the workflow of the cartridge, the workflow of the ECL bioassay, and the workflow of analysis method.

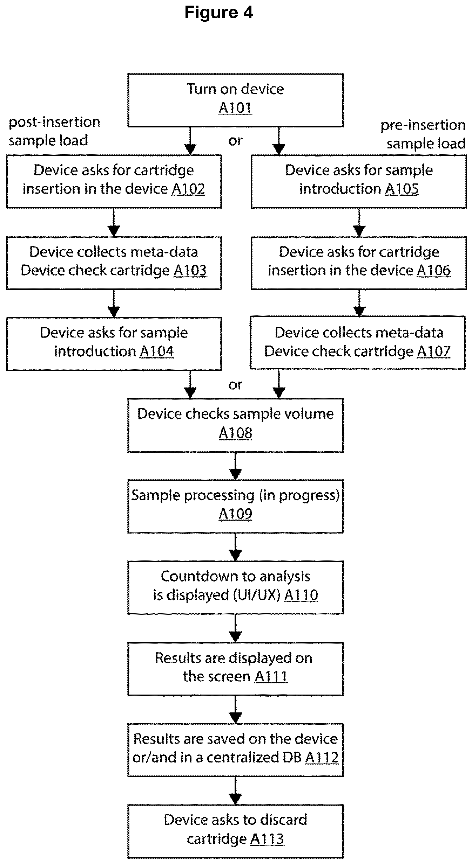

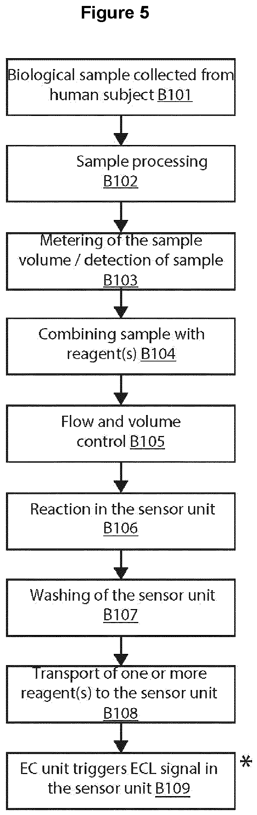

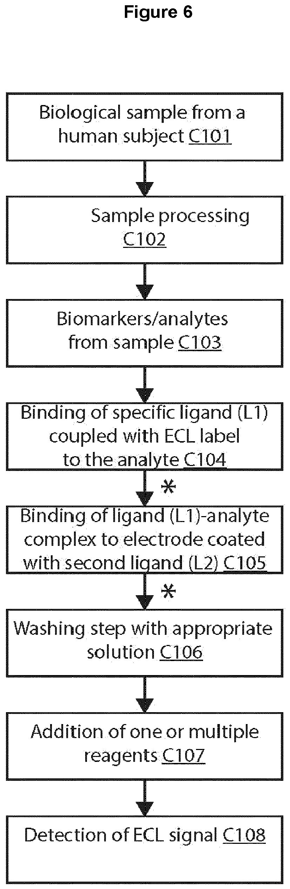

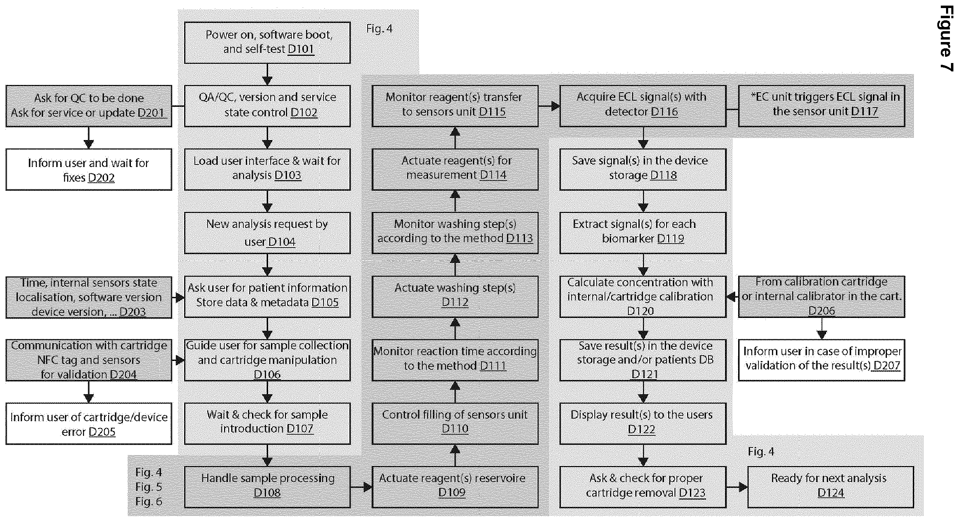

Figures 4 to 7 show an embodiment of the invention which is the workflows of the diagnosis method carried out in the device (Fig. 4), the cartridge (Fig. 5) the ECL bioassay (Fig. 6), and the analysis method (Fig.7).

The method shown in Figure 4 is the workflow caried out in the base station, also called the reader device and comprises the following steps:
First, of course, the device has to be turned on A101, followed by a post-insertion sample load or pre-insertion sample load:
- in the case of post-insertion sample load, which means that the cartridge is inserted into the device and afterwards the sample is added to the cartridge, the device will ask for cartridge insertion in the device A102, then the device will collect meta-data and check the cartridge A103, and then will ask for the sample introduction A104. An advantage of post insertion is that it permits for example to have the cartridge preconditioned (e.g. pre-heating) prior sample introduction.
- in the case of pre-insertion sample load, which means that the sample is added to the cartridge and afterwards the cartridge is inserted into the device, the device will ask for sample introduction A105, then the device will ask for cartridge insertion A106, then the device will collect meta-data and check the cartridge A107; An advantage of pre-insertion permits for example to shake before insertion and therefore improve mixing.

Once done, the device first determines the sample volume A108 thanks to a metering which can be a capillary or a volumetric valve or a membrane and carries out a sample processing A109. The sample processing may comprise at least one of, but is not limited to, a serum extraction phase on a blood filtration membrane, addition of buffer, metering via a capillary or a volumetric valve, a pre-concentration step on a solid phase, addition of magnetic beads, and the same.

Optionally, a countdown to analysis is displayed (U I/UX) A110; and once obtained the results are shown on the display A111 and preferably saved on the device or/and in a centralized data base A112. Finally, the device asks to disengage and discard the cartridge A113.

The method shown in Figure 5 is the cartridge workflow that is implemented using the base station. After the cartridge receives the biological sample B101, all operations B102-B109 can be performed on-cartridge, with the device software partially or fully controlling all on-cartridge operations. The workflow may be comprised of the following steps:
The first step comprises obtaining a biological sample from a human subject B101. This can be done through capillary collection tube or deposition of a drop of biological fluid (e.g., whole blood, serum, etc.) from a pipet, for example. Then in a second step, the method performs the sample processing steps B102 such as the separation of plasma from whole blood to assess the TBI/mTBI biomarkers potentially present in the biological sample and then meters the sample volume while starting the detection of the sample presence in the cartridge B103.

If a sample presence is detected, then in a next step, the method starts combining the sample with the assay reagent(s), for instance, biomarker(s)-specific ligands (L1) coupled with electrochemiluminescent (ECL) detection label(s) (L1/ECL) B104 to form biomarker(s)-L1/ECL complex(es), while controlling the flow and volume B105. That means ligands for one or several different biomarkers. These ligands can be, but not limited to antibodies, aptamers, adhirons, and the same.

Biomarker(s)-L1/ECL complex(es) are then transferred to the sensor(s) unit B106 where they can react with biomarker(s)-specific ligands (L2) pre-functionalized on the electrode surface. After an incubation time, a washing step of the sensor(s) unit B107 is caried out and followed by the transport of one or more reagents (that might include ECL buffers) to the sensor unit B108.

Finally, detection of biomarker(s) is carried out where one is applying potential (from EC unit 120, see Figure 1) to the sensor unit to trigger ECL read-out B109.

The method shown in Figure 6 is a description of the ECL bioassay workflow that is carried out in the cartridge and comprise the following steps:
(a) obtaining a biological sample from a human subject C101 and performing the sample processing steps C102 to assess TBI/mTBI biomarker(s) potentially present in the biological sample C103;
(b) binding of one or a plurality of TBI/mTBI biomarker(s) C103 potentially present in the biological sample with biomarker(s)-specific ligands (L1) coupled with an electrochemiluminescent (ECL) detection label (L1/ECL) to form a biomarker(s)-L1/ECL complex(es) C104. Examples of biomarkers are, but not limited to, GFAP (glial fibrillary acidic protein), h-FABP (heart-fatty acidic binding protein), IL-6 (Interleukin-6), IL-8 (Interleukin-8), IL-10 (Interleukin-10), NFL (neuro filament light), NSE (neuron-specific enolase), S100b (S100b calcium binding protein), UCH-L1 (ubiquitin C-terminal hydrolase), and examples of ligands include antibodies, aptamers, adherons, and examples of labels are luminol, ruthenium (II), osmium (II), platinum (II) or Ir(III)-based complexes, metal oxides (TiO2, SnO2, ZnO);
(c) binding of biomarker(s)-L1/ECL complex(es) to the conductive electrode surface (in the sensor unit) functionalized with a plurality of biomarker(s)-specific ligands (L2) to form a sandwich immunoassay complex(es) (L1/ECL-biomarker(s)-L2) C105, wherein each of the ligands L2 can be immobilized at a different, separate locations on the conductive electrode surface;
(d) washing step C106;
(e) addition of the ECL buffer solution C107;
(f) detection of ECL signal by applying potential to the sensor unit to trigger ECL read-out and correlating it to the concentration of TBI/mTBI biomarker(s) present in the biological sample C108.

The invention also relates to a computer implemented method which controls the cartridge process and the acquisition of the signal by the detector (detector unit 130, see Fig. 1) once the ECL signal is triggered by the potentiostat (EC unit 120, see Fig. 1) to the electrodes. The acquired signal is then processed to extract a plurality of specific responses for each analyzed biomarkers, calculates a concentration by using an internal or cartridge-based (external) calibration curve and provides the results to the user. At the same time, those results, including the meta-data and the raw image data are stored in the internal memory or/and remotely in a centralized database.

Figure 7 provides a detailed description of the workflow of the computer implemented method comprised of the following tasks:
(a) power on, software boot, and self-test D101;
(b) QA/QC, version and service state control D102 in other words, the software asks for QC to be done/ask for service, maintenance or update D201; software informs the user and waits for fixes D202;
(c) load user interface and wait for analysis D103;
(d) new analysis/measurement request by the user D104;
(e) ask user for patient information, store information & metadata D105 (time, internal sensors state localization, software version, device version D203);
(f) guide user for sample collection and cartridge manipulation D106 (communication with cartridge NFC tag and sensor for validation D204; inform user of cartridge/device error D205);
(g) wait & check for sample introduction D107;
(h) handle sample processing tasks D108;
(i) actuate reagent(s) reservoir(s) D109;
(j) control filing of sensor unit D110;
(k) monitor reaction time according to the method D111;
(l) actuate washing step(s) D112;
(m) monitor washing step(s) according to the method D113;
(n) actuate reagent(s) for measurements D114;
(o) monitor reagent(s) transfer to sensor unit D115;
(p) acquire ECL signal(s) with detector D116 (*in parallel EC unit triggers ECL signal in the sensor unit D117);
(q) save signal(s) data in the device storage D118;
(r) extract signal(s) data for each biomarker D119;
(s) calculate biomarker(s) concentration(s) with internal/cartridge calibration D120 (from calibration cartridge or internal calibrator in the cartridge D206, and inform user in case of improper validation of the results D207);
(t) compare the calculated biomarker(s) concentration(s) with a predetermined threshold, which can be a discrete value or a range and estimates a diagnosis trend on that basis, for example, if the concentration is by far higher than the threshold one estimates that the patient is TBI positive while if the concentration is slightly above, one estimates that it necessitates a second test later, and so on.
(u) save result(s) in the device storage and/or patient's database D121;
(v) display result(s) to the user D122;
(w) ask & check for proper cartridge removal D123 (ready for next analysis D124).

According to a preferred embodiment, the signal of each biomarker is extracted from the image (signal) by the software (detection and alignment of spots on the sensors, detection of controls spots and identification of spots based on the array template) to obtain the intensity of each biomarker in the microarray on the sensors. Controls spots intensities are evaluated to perform a quality control (QC) based on a threshold signal. Once this control is successfully done, intensity of each biomarker is further process with a cartridge based or internal calibration curve to obtain the concentration in the patient blood and provided as ng·mL⁻¹, pg·mL⁻¹ or any other clinically meaningful concentration units. When biomarkers level or Controls signals are out of the calibration or control range the device informs the user of improper results and invalidates the test results. The concentration of biomarkers could be compared to a clinically meaningful cut-off (threshold) values to support diagnostic evaluation by a healthcare professional.

While the embodiments have been described in conjunction with a number of embodiments, it is evident that many alternatives, modifications and variations would be or are apparent to those of ordinary skill in the applicable arts. Accordingly, this disclosure is intended to embrace all such alternatives, modifications, equivalents, and variations that are within the scope of this disclosure. This for example particularly the case regarding the different apparatuses which can be used.

## Claims

1. Analytical cartridge for sample processing and quantitative multi-analyte immunoassay comprising
a sample inlet port (201),
at least one reagent retaining element (204),
a mixing unit (206) where the sample and the reagents are combined,
a sensor unit (207) comprising electrodes pre-functionalized with biomarker(s)-specific ligands, where the mixed solution is reacted with pre-functionalized ligands,
a reagent reservoir unit (211) adapted to dispense an ECL buffer into the sensor unit prior measurement, and **characterized in that**
the sensor unit is adapted to be powered by an electrical power supply source adapted to apply a potential to the pre-functionalized electrodes and trigger an ECL reaction between the reagents producing an ECL signal to be acquired by a detector unit (130).

2. Analytical cartridge according to claim 1, **characterized in that** it is a cartridge for quantitative multi-analyte immunoassay for TBI and/or mTBI diagnostics.

3. Analytical cartridge according to claim 1 or 2, **characterized in that** the sample inlet port (201) is preferably designed to collect a volume between 10 to 100 µL.

4. Analytical cartridge according to any one of claims 1 to 3, **characterized in that** the reagent reservoir(s) is adapted to remove non-specifically bound compounds in the sensor unit and to dispense an ECL buffer into the sensor unit prior measurement or **in that** it further comprises a wash buffer unit (208) adapted to remove the non-specifically bound compounds in the sensor unit.

5. Analytical cartridge according to any one of claims 1 to 4, **characterized in that** it further comprises a sample processing unit (202) comprising at least one of a filtration unit or a microfluidic blood separator system, and a metering device (203) adapted to support accurate quantification of the biomarkers, and comprising at least one of an active valve, a passive microfluidic channel, and/or a membrane.

6. Analytical cartridge according to any one of claims 1 to 5, **characterized in that** at least one reagent retaining element (204) is adapted to store reagents immobilized, lyophilized, or in solution.

7. Analytical cartridge according to any one of claims 1 to 6, **characterized in that** at least one reagent retaining element (204) is adapted to be actuated by an integrated pumping module or by applying manually or mechanically pressure on a pouch (205).

8. Analytical cartridge according to any one of claims 1 to 7, **characterized in that** the reagents solution stays in the sensor unit during an incubation time of between 1 to 30 min.

9. Analytical cartridge according to any one of claims 1 to 8, **characterized in that** it comprises a transparent cartridge window (217).

10. Point-of-Care in vitro diagnostic (IVD) medical device for quantitative multi-analyte immunoassay comprising the analytical cartridge according to any one of claims 1 to 9 and a base station, wherein the base station comprises
a cartridge interface (102) for connecting to the cartridge,
electronics for managing the tasks of analysis/measurement and a user interface (UI) (173) comprising one or multiple display(s) (171) and buttons/keyboard/touch screen (172),
actuators (140) for activating the fluids in the cartridge through specific connectors on the device (141) and on the cartridge (142),
an electrochemistry unit (120) comprising an electronic hardware (121) employed for an ECL signal trigger in sensor unit of the cartridge (207), via an interface (123),(124), said trigger unit comprising an electrical power supply source adapted to apply a potential to the sensor unit through the pre-functionalized electrodes and trigger ECL reaction between the reagents,
a detector unit (130) for detecting ECL signal generated in the sensor unit (207) of the cartridge (101) composed of a detector (131) and connected via an optic path (132) to a window (133) interfacing with the cartridge (101) via a window on the cartridge (134), and
a communication unit (110) adapted to provide connectivity to the cartridge with an NFC reader (112) to access cartridge data and ID and a Network (5G/WIFI/BT) (111) unit for database connections, software updates and general communication.

11. Biomarker detection method comprising the steps of:
- obtaining a biological sample from a human subject and performing the sample processing steps to assess TBI and mTBI biomarker(s) potentially present in the biological sample;
- binding of one or a plurality of TBI and mTBI biomarkers present in the biological sample with biomarker(s)-specific ligands coupled with an electrochemiluminescent detection label (L1/ECL) to form a biomarker(s)-L1/ECL complex(es);
- binding of said biomarker(s)-L1/ECL complex(es) in the sensor unit to the conductive electrode surface functionalized with a plurality of biomarker(s)-specific ligands (L2) to form a sandwich immunoassay complex(es) (L1/ECL-biomarker(s)-L2), wherein each of the ligands L2 can be immobilized at a different, separate location on the conductive electrode surface;
- washing step;
- adding an ECL buffer solution;
- detecting of ECL signal for each biomarker by applying potential to the sensor unit to trigger ECL read-out and correlating it to the concentration of TBI/mTBI biomarker(s) present in the biological sample.

12. A method according to claim 11, wherein the electrode's surface can be functionalized with one or plurality of biomarker(s) specific ligands (L2), on one or multiple, different, indexable, locations on the electrode.

13. A method according to claims 11 or 12, wherein electrochemiluminescent (ECL) detection labels are loaded on carrier particles, taken in the group comprising mesoporous, conductive, magnetic and nano/microparticles, wherein the carrier particles carrying the label are coupled to biomarker(s)-specific ligands (L1).

14. A method according to claims 11-13, wherein the electrode is modified with a multi-purpose functionalized interface layer wherein the layer is formed from various materials and exhibits and/or improves one or multiple properties, such as:
- antifouling (reduces background interference and/or non-specific binding);
- enrichment (target analyte/antibody/ECL detection label enrichment and/or antigen antibody binding events/kinetics improvement);
- conductivity (improves electron transfer and ECL signal intensities);
- wettability (improves wettability of the electrode and liquid flow).

15. TBI and mTBI diagnosis method comprising the biomarker(s) detection method of anyone of claims 11 to 14 and further comprising the steps of:
- determining whether the TBI and mTBI biomarker(s) concentrations are above or below a predetermined threshold range;
- based on the determining step, judging that TBI and mTBI is diagnosed if the calculated concentrations are above said threshold ranges;
- displaying results to the user for IVD TBI and mTBI diagnosis;
- saving results for kinetic (i.e., multiple follow-up) analyses (monitoring) of biomarkers over time with multiple sampling of the same patient. Evaluation of the kinetics to support diagnosis of TBI or mTBI.

16. Method according to claims 11-15, further comprising the step of the saving results in a patient's database comprising data corresponding to biomarker(s) measurements performed at different time points with respect to the target marker(s) and wherein the data and background information related to the patient comprises demographic information corresponding to at least one of age, race, gender, body mass index, and morbidities.
